# EUROPÄISCHE PATENTSCHRIFT

(11) **EP 0 630 402 B1**
(45) Veröffentlichungstag und Bekanntmachung des Hinweises auf die Patenterteilung: **04.06.1997**
(21) Anmeldenummer: 93903849.3
(22) Anmeldetag: 05.03.1993
(51) Int. Cl.: C12N 9/02, C12P 7/00

(54) **NEUE KETOESTER-REDUKTASE, DEREN HERSTELLUNG UND VERWENDUNG FÜR ENZYMATISCHE REDOXREAKTIONEN**
NEW KETONIC ESTER REDUCTASES, ITS PREPARATION AND USE FOR ENZYMATIC REDOX REACTIONS
NOUVELLE REDUCTASE D'ESTERS CETONIQUES, FABRICATION ET UTILISATION POUR DES REACTIONS REDOX ENZYMATIQUES

(30) Priorität: 13.03.1992 DE 4207921
(43) Veröffentlichungstag der Anmeldung: 28.12.1994
(73) Patentinhaber: FORSCHUNGSZENTRUM JÜLICH GMBH, 52425 Jülich (DE)
(72) Erfinder: KULA, Maria-Regina, D-5162 Niederzier (DE); PETERS, Jörg, D-4006 Erkrath 1 (DE)
(86) Internationale Anmeldenummer: DE9300198
(87) Internationale Veröffentlichungsnummer: WO9318138

(56) Entgegenhaltungen:
- DIALOG INFORMATION SERVICES, File 155 Medline, Dialog AN 02804302; J.M. UTTING et al., AN 75211302
- DIALOG INFORMATION SERVICES, File 5 BIOSIS, Dialog AN 10061149; J. PETERS et al., AN 95061149

## Beschreibung

Gegenstand der Erfindung ist eine neue zur NADH-abhängigen enzymatischen Umsetzung von β-, γ- und δ-Ketoestern zu den entsprechenden optisch aktiven β-, γ- und δ-Hydroxysäureestern befähigte Ketoester-Reduktase, die aus Stämmen von *Candida parapsilosis, Yarrowinia cellobiosa, Rhodococcus erythropolis* oder *Pseudomonas acidovorans* isolierbar ist.

Optisch aktive β-, γ- und δ-Hydroxycarbonsäureester sind wertvolle chirale Synthesebausteine, die eine breite Anwendung in der Synthese von Pharmaka, Aromastoffen, Pheromonen, Agrochemikalien und Enzym-Inhibitoren finden. Sie sind auf konventionellem chemischem Wege nur schwer zugänglich, da die Trennung des durch chemische Reduktion entstehenden Enantiomerengemisches aufwendig und kostenintensiv ist.

Bekannt sind Arbeiten zur fermentativen Herstellung von β-, γ- und δ-Hydroxycarbonsäuren und -estern mit Hilfe von Mikroorganismen. Dabei werden die mikrobiellen Zellen stets im Überschuß eingesetzt und die Ausbeute und Enantiomerenüberschüsse variieren je nach Herkunft der Zellen über einen weiten Bereich. Am häufigsten wird dabei die Bäckerhefe *Saccharomyces cerevisiae* eingesetzt.

Bekannt sind auch bereits aus *Saccharomyces cerevisiae* isolierte Oxidoreduktasen, welche die enzymatische Reduktion der β-, γ- und δ-Ketogruppe von entsprechenden Ketoestern unter Bildung der entsprechenden optisch aktiven Hydroxyverbindungen katalysieren (Heidlas et al. (1988) Eur. J. Biochem. 172: 633-639). Diese bekannten Oxidoreduktasen benötigen NADPH als Cokatalysator, dessen Regenerierung diffizil ist, weshalb die gewerbliche Akzeptanz dieses an sich bekannten enzymatischen Syntheseweges nicht ohne weiteres gegeben ist.

Ziel der Erfindung war daher eine durch NADH cokatalysierte enzymatische Umsetzung von β-, γ- und δ-Ketoestern sowie ein dafür geeignetes Enzym.

Dieses wurde überraschenderweise in bestimmten Hefen und Bakterien gefunden, und zwar in *Candida parapsilosis* und *Yarrowinia cellobiosa* sowie in *Rhodococcus erythropolis* und *Pseudomonas acidovorans,* die in der Lage sind *n*-Alkane bzw. *n*-Alkansäure zu verwerten. Die aus *Candida parapsilosis* und *Rhodococcus erythropolis* isolierte Ketoester-Reduktase wurde umfangreich untersucht.

Besonders hohe Aktivitäten der neuen Ketoester-Reduktase (KERed) wurden erzielt, wenn *Candida parapsilosis* bzw. *Rhodococcus erythropolis* auf einem Nährmedium gezüchtet wurden, das längerkettige Alkansäuren bzw. Alkane, und zwar insbesondere Dodecansäure und Tetradecan, als Kohlenstoff-Quelle enthielt.

Besonders intensiv wurde die Bildung der KERed mittels *Candida parapsilosis* DSM 70 125 und die Isolierung und Aufreinigung des Enzyms aus diesem Stamm untersucht.

Die gereinigte KERed zeichnet sich durch folgende Parameter aus :
- ein pH-Optimum für die Ketoester-Reduktion zwischen pH 7,8 und 8,0 und für die Rückreaktion um pH 9,5.
- ein Temperatur-Optimum für die Ketoester-Reduktion zwischen 36°C und 40°C und für die Rückreaktion von 50°C bis 56°C.
- rasche Desaktivierung durch Hg²⁺-, Pb²⁺-, Ag⁺-, Cu²⁺-, Ni²⁺-, Sn²⁺- und Co²⁺-Ionen. Starke Inhibierung durch p-Hydroxymercuribenzoat, 5.5'Dithio-Bis(2-Nitrobenzoat) und Jodacetamid sowie druch die Chelatoren 2.2'-Bipyridyl und o-Phenanthrolin. Stabilisierung durch SH-Schutzreagenzien wie Dithiothreitol.
- zusätzliche Befähigung zur Reduktion von aliphatischen, alicyclischen und aromatischen Ketonen, Diketonen, Ketalen und Aldehyden sowie zur Oxidation von primären und sekundären Alkoholen

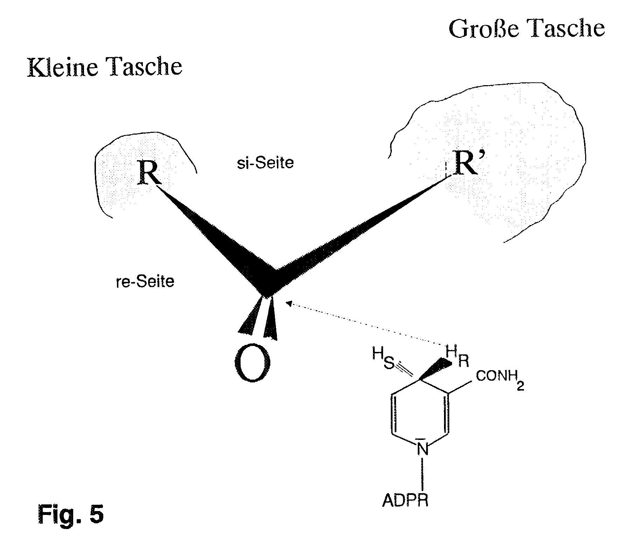

Die reduktive enzymatische Umsetzung von β-, γ- und δ-Ketoestern zu den entsprechenden optisch aktiven Hydroxycarbonsäureestern erfolgt nach folgender Gleichung : 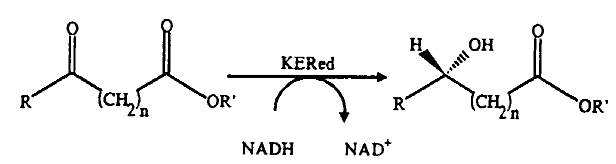 R und R' können Reste breiter Vielfalt sein wie aus Tabelle 4 hervorgeht. n kann Werte zwischen 1 und 3 annehmen. Darüberhinaus skizziert das Modell der Substratbindungsstelle (Fig. 5, Tabelle 5, Beispiel 3.G) die Grenzen der Substratakzeptanz.

Neben den Ketoestern werden von der KERed weitere Oxoverbindungen als Substrate akzeptiert, wie insbesondere 1,1-Dichloraceton, Chloraceton, 3-Chlor-2-Butanon, 2-Octanon, 2-Butanon, 2-Pentanon, 2-Methyl-3-Pentanon, Methoxy-2-Propanon, Acetophenon, 3-Chloracetophenon, 4-Fluoracetophenon, 4-Bromacetophenon, Propiophenon, 2,4-Hexandion, 2,4-Pentandion sowie Acetaldehyd, Isobutyraldehyd und Propionaldehyd. Acetophenon wird zu (S)-Phenylethanol, 3-Oxobuttersäureethylester zu (*S*)-3-Hydroxybuttersäureethylester und 5-Oxohexansäureethylester zu (S)-5-Hydroxyhexansäureethylester umgesetzt.

Interessant ist in diesem Zusammenhang speziell die Möglichkeit der Umsetzung von Acetophenon, 4-Bromacetophenon, 3-Chloracetophenon und 4-Fluoracetophenon zu den entsprechenden (S)-Phenylethanolen in NADH-Cokatalyse, da bislang nur die (R)-spezifische Umsetzung realisiert ist.

Die Rückreaktion (Oxidation der Hydroxygruppe) ist ebenso enzymatisch durchführbar und kann insbesondere dazu ausgenutzt werden, durch Umsetzung des S-Enantiomeren eines Racemats von R- und S-Hydroxyverbindungen das ggf. gewünschte R-Enantiomere zu gewinnen.

Die nachstehend im einzelnen beschriebene Ketoester-Reduktase ist, wie dargelegt, nicht nur zur Reduktion der β-, γ-, δ-Ketogruppen der Ketoester befähigt, sondern darüberhinaus zur Reduktion einer breiten Vielfalt von Carbonylverbindungen verwendbar. Daher kann das Enzym auch als Carbonyl-Reduktase bezeichnet werden.

Das neue Enzym kann innerhalb der EC-Klassifizierung unter der Nummer [EC 1.2.1] eingeordnet werden. Eine genaue Zuweisung einer EC-Nummer steht bisher allerdings noch aus. Der Einfachheit halber wird das neue Enzym in der anschließenden Beschreibung als Ketoester-Reduktase (KERed) bezeichnet.

Man erhält die KERed in an sich bekannter Weise durch Kultivierung (in üblichen Nährmedien ggf. in Gegenwart von Alkanen und/oder Alkansäuren) der genannten Mikroorganismen, aus deren Rohextrakt ein verwendbares Enzympräparat durch fraktionierte PEG-Fällung erhalten werden kann, bei der zunächst mit relativ niedermolekularem Polyethylenglycol (PEG) für die Ausfällung von Begleitproteinen gesorgt wird, während die KERed noch in Lösung bleibt, die dann aus der Flüssigkeit durch PEG mit erhöhtem Molekulargewicht ausgefällt und aus dem Niederschlag durch Pufferlösung wieder "extrahiert" wird.

Alternativ kann mit unterschiedlichen PEG-Konzentrationen aber gleichbleibendem Molekulargewicht im Bereich von 1.000 bis 10.000, insbesondere um 5.000, gearbeitet werden. Eine weitere Reinigung durch chromatographische Trennmethoden führt zu bevorzugten Isolaten mit höherer spezifischer Aktivität wie anhand des nachfolgend wiedergegebenen Beispiels dargelegt wird.

Im Folgenden wird die Erfindung mehr im einzelnen anhand von Beispielen beschrieben. Dabei wird auf die angefügten Zeichnungen Bezug genommen. Es zeigen :

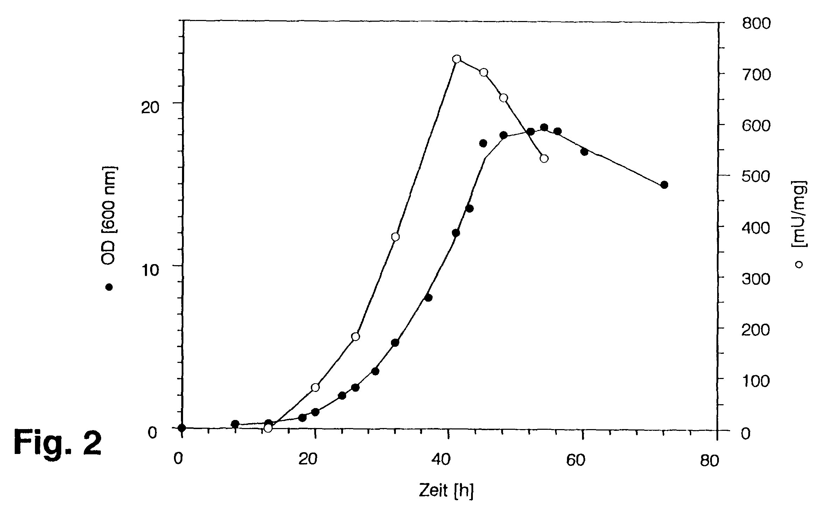

- Figur 1 und 2: die Bildung der KERed mittels *C. parapsilosis* bei Wachstum auf Glycerin (1) bzw. Dodecansäure (2)
- Figur 3: die Regulation der Aktivität der KERed aus *C. parapsilosis* a) 5.4% Glucose, b) 0.8% Glucose, c) 2% Glycerin, d) 2% Glycerin + 0.1% Induktor, e) 1% Dodecansäure, f) 1% Dodecansäure + 0.1% Induktor; Induktor : 3-Oxohexansäureethylester
- Figur 4: die pH-Abhängigkeit der KERed-Aktivität bei Oxidation und Reduktion
- Figur 5: Modell der Substratbindungsstelle der KERed aus *C. parapsilosis*
- Figur 6: die chromatographische Trennung von (*R*)- und (*S*)-3-Hydroxybuttersäuremethylester und zwar (a) für das racemische Gemisch und (b) für das erfindungsgemäße KERed-Umsetzungsprodukt
- Figur 7: die chromatographische Trennung von (*R*)- und (*S*)-5-Hydroxybuttersäureethylester für das erfindungsgemäße KERed-Umsetzungsprodukt

### Beispiel 1 : Gewinnung des Enzyms

### 1. Screening

Sammlungsstämme der DSM (Deutsche Sammlung von Mikroorganismen und Zelkulturen GmbH, Braunschweig) wurden in dem von der DSM empfohlenen Medium angezogen, die Zellmasse durch Zentrifugation geerntet und durch Naßvermahlung aufgeschlossen. Der Überstand diente als Enzymquelle (Rohextrakt) und wurde auf Ketoester-Reduktase-Aktivität untersucht. Der Test wurde photometrisch durchgeführt, wobei der Testansatz folgende Komponenten enthielt :
- 0,2 mM NAD(P)H
- 8 mM Ketoester
- 0,1 M Triethanolamin (TEA)-NaOH-Puffer, pH 7
- limitierende Mengen an Rohextrakt

Es wurde die NAD(P)H-Abnahme bei 334 nm über einen Zeitraum von 1 Minute verfolgt. Zur Aktivitätsberechnung wurde ein Absorptionskoeffizient von 6,18 M⁻¹cm⁻¹ verwendet. Tabelle 1 faßt die erhaltenen spezifischen Aktivitäten für die einzelnen Mikroorganismen zusammen, die sich aus einer Gruppe von 65 getesteten Mikroorganismen als tauglich erwiesen haben.

Pro Mikroorganismus ist in der ersten Zeile die NADH-abhängige, in der zweiten Zeile die NADPH-abhängige spezifische Aktivität in mU/mg angegeben. Ein U (Unit) entspricht einem Umsatz von 1 µmol reduziertem Coenzym pro Minute.

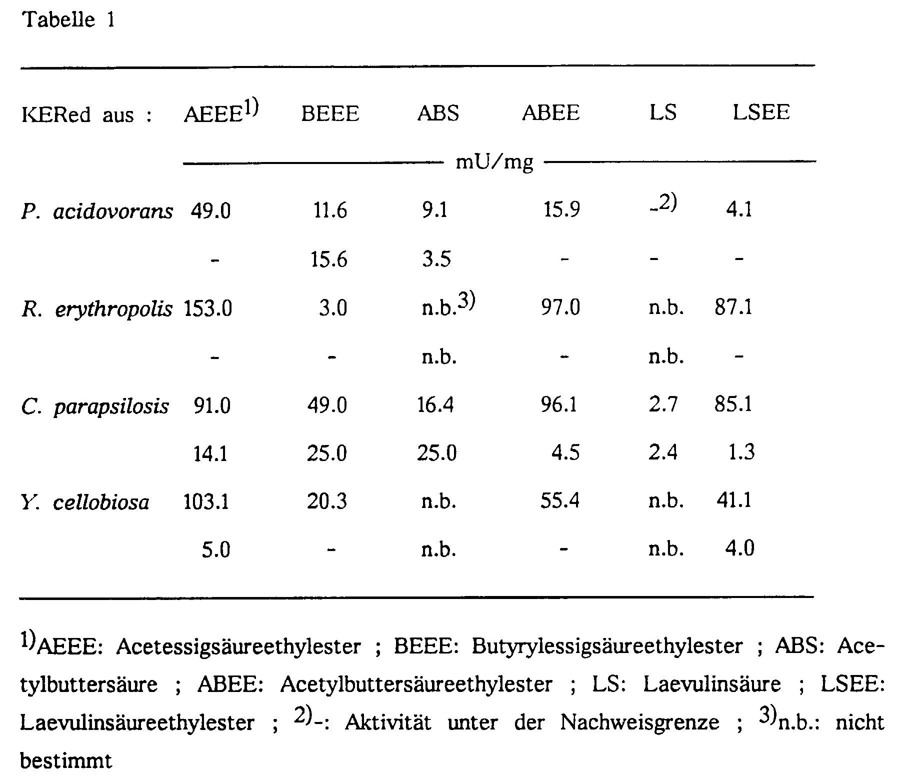

### 2. Züchtung von Candida parapsilosis

Zur Enzymgewinnung wurde *Candida parapsilosis* in folgendem Medium angezogen (pro 11) :

| | | |
|---|---|---|
| Hefeextrakt | 10 g | |
| Kaliumphosphat | 1 g | |
| Ammoniumsulfat | 1.2 g | |
| Glycerin | 20 g | (nicht reprimiert) |
| oder Glucose | 54 g | (reprimiert) |
| oder Glucose | 8 g | (dereprimiert) |
| oder Dodecansäure | 10 g | |

ggf. 0,1%iger Zusatz von 3-Oxohexansäureethylester (Induktor)

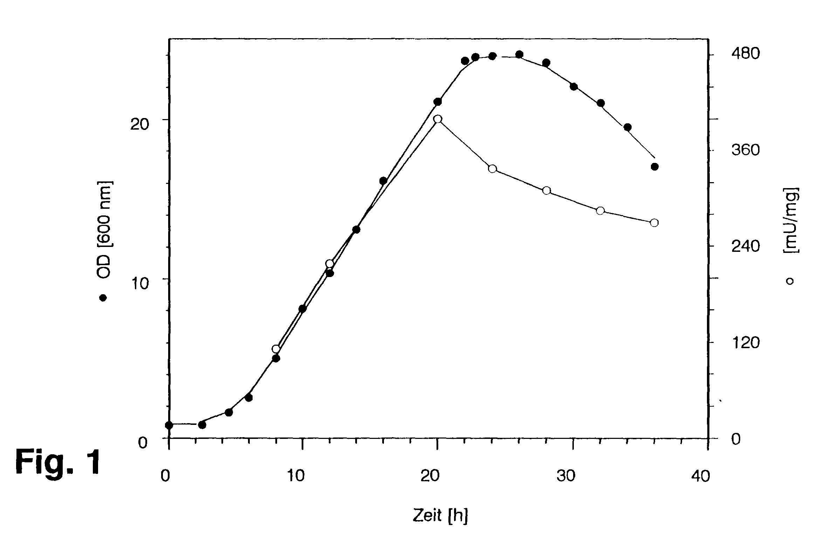

Der pH-Wert dieser Lösung wurde auf 4,5 eingestellt, dann wurde für 15 min bei 121°C (2 bar) sterilisiert. Der Organismus wurde aerob kultiviert. Im 10 1-Maßstab wurde das Medium nach Erreichen der Bruttemperatur von 30°C mit 400 ml einer 20 Std. alten Vorkultur beimpft. In einem solchen 10 1-Ansatz wurde beispielhaft der Verlauf der Enzymaktivität über der Zeit bestimmt, indem Proben zu verschiedenen Zeiten entnommen wurden und die Aktivität der Ketoester-Reduktase nach Aufschluß der Zellen bestimmt wurde. In Fig. 1 ist ein solcher Verlauf dargestellt, die Aktivität der Ketoester-Reduktase erreicht nach 20 Std. einen maximalen Wert und fällt anschließend wieder ab. Die Zellernte erfolgte nach 20 Stunden durch Zentrifugation, wobei aus 8 1 Medium 175 g Feuchtmasse erhalten wurden. Die Zellmasse kann bei -20 °C eingefroren gelagert werden, wobei über mehrere Wochen kein Aktivitätsverlust erkennbar ist.

### 3. Enzymisolierung (Rohextrakt)

Die Enzymfreisetzung aus den ganzen Zellen kann durch an sich bekannte Methoden (Ultraschall, Hochdruck-Homogenisation, Naßvermahlung u.a.) geschehen. Hier wurden die Zellen durch Naßvermahlung mit Glasperlen aufgeschlossen. Dazu wurde die Zellmasse (175 g) in Aufschlußpuffer (200 mM TEA-NaOH-Puffer (pH 7,5) unter Zusatz von 5 mM Dithiothreitol und 1 mM Protease-Inhibitor (Pefabloc, Fa. Merck, Darmstadt) suspendiert, so daß die Konzentration der Zellfeuchtmasse 25%ig war (700 ml Endvolumen).

Die Zellinhaltsstoffe wurden aus der gekühlten Suspension (4°C) durch einen mechanischen Aufschluß mit Hilfe einer Glasperlenmühle (Desintegrator S, Fa. IMA, Frankfurt) freigesetzt. Der Mahlbehälter wurde dazu mit Glasperlen (0,5 mm : 0,3 mm = 2 : 1 - Verhältnis, 90 ml) gefüllt und mit 60 ml der 25%igen Zellsuspension aufgefüllt. Der Aufschluß wurde bei einer Rührwellendrehzahl von 4000 UpM durchgeführt. Der Kühlmantel wurde während des Laufs gekühlt. Eine Aufschlußzeit von 9 min erwies sich als optimal und wurde für den Zellaufschluß verwendet. Nach dem Zellaufschluß wurde der Überstand dekantiert und die Glasperlen zweimal mit Aufschlußpuffer gewaschen.

175 g Hefefeuchtmasse ergaben 285 ml Rohextrakt mit einer Volumenaktivität von 6,6 U/ml und einem Proteingehalt von 10,7 mg/ml. Hieraus läßt sich errechnen, daß aus 100 1 Fermenteransatz ca. 23 000 Einheiten Ketoester-Reduktase gewonnen werden können (1 Enzymeinheit ist die Enzymmenge, die 1 µmol Acetylbuttersäureethylester pro Minute umsetzt).

### 4. Anreicherung des Enzyms

Das Enzym kann durch an sich bekannte Methoden der Proteinreinigung wie zweistufige, fraktionierte Polyethylenglycol-Fällung, Ionenaustausch-Chromatographie und Affinitätschromatographie angereichert und gereinigt werden.

### 4.1 Fraktionierte Polyethylenglycol-Fällung

Eisgekühlter Rohextrakt (4°C) wird unter Rühren und pH-Kontrolle (pH 7-7,5) mit Polyethylenglycol (M_{w}: 5000) bis zu einer Konzentration von 4% (w/v) versetzt. Nach Zentrifugieren bei 10 000 UpM für 10 min wird der Überstand weiter mit PEG₅₀₀₀ bis zu einer Endkonzentration von 30% versetzt. Das nach Zentrifugation bei 10 000 UpM für 15 min erhaltene Sediment enthält die Ketoester-Reduktase-Aktivität und kann in Triethanolamin-NaOH-Puffer, 20 mM, pH 7,5 aufgenommen und lyophilisiert werden. Das Enzym ist unter diesen Bedingungen mehrere Monate ohne Aktivitätsverlust haltbar.

### 4.2 Ionenaustausch-Chromatographie

1 ml resuspendiertes Sediment aus der 30%igen PEG-Fällung (entspr. Beisp.1; 4.1) wird auf eine Q-Sepharose-FastFlow-Säule (Anionen-Austauscher, HiLoad, High Performance, 16/10, 20 ml Gelvolumen) aufgetragen (FPLC-Chromatographie-System der Fa. Pharmacia, Freiburg). Die Säule ist mit Puffer A äquilibriert worden (20 mM Triethanolamin-NaOH-Puffer; pH 7,5). Nach ausgiebigem Waschen der Säule mit Puffer A wird die Ketoester-Reduktase mit einem linearen NaCl-Gradienten (0-200 mM) eluiert, wobei das Enzym bei ca. 100 mM NaCl eluiert. Die Chromatographie wurde bei einer Flußrate von 1 ml/min durchgeführt. Die erzielte Anreicherung ist in Tab. 2 zusammengefaßt.

### 4.3 Affinitätschromatographie

Die Fraktionen der Q-Sepharose-Chromatographie mit der höchsten Aktivität werden gesammelt, mit Hilfe einer Ultrafiltrationszelle (Amicon, Witten/Ruhr) aufkonzentriert und weiter aufgereinigt durch Chromatographie an Agarose-NAD (Fa. Sigma, Deisenhofen). Es wurde das Niederdruck-Chromatographie-System der Fa. Pharmacia verwendet. Das Gelbett betrug 5 ml. Die Chromatographie wurde mit einer Flußrate von 0,5 ml/min durchgeführt. Die Anreicherung ist in Tab. 2 zusammengefaßt.

**Tabelle 2 :**

| Anreicherung der Ketoester-Reduktase | | | |
|---|---|---|---|
| Reinigungsschritt | Spez.-Aktivität (U/mg) | Gesamt-Ausbeute (%) | Anreicherung (-fach) |
| Rohextrakt | 0,6 | 100 | 1,0 |
| 4% PEG-Fällung | 0,7 | 75 | 1,2 |
| 30% PEG-Fällung | 2,5 | 75 | 4,2 |
| Q-Sepharose FF | 40,3 | 71 | 67,0 |
| Ultrafiltration | 40,3 | 67 | 67,0 |
| Agarose-NAD⁺ | 1855,0 | 67 | 3091,0 |

Das scheinbare native Molekulargewicht der Ketoester-Reduktase von 136 kDa (± 11 kDa) wurde durch Gelperrneations-Chromatographie an Sephadex G-200 bestimmt. Dabei dienten Thyroglobulin, Ferritin, Katalase, Aldolase, Rinderserumalbumin, Ovalbumin, Chymotrypsinogen A und Ribonuclease A als Eichproteine. Die beiden gleich großen Untereinheiten haben ein scheinbares Molekulargewicht von 67 kDa, wie durch SDS-Gelelektrophorese bestimmt wurde. Als Molekulargewichtsstandard wurde eine Mischung aus α₂-Makroglobulin, Phosphorylase b, Rinderserumalbumin, Glutamat-Dehydrogenase, Lactat-Dehydrogenase und Trypsininhibitor verwendet.

Je nach unterschiedlicher Herkunft der KERed sind gewisse Abweichungen im Molekulargewicht bei gleichbleibender Funktion (NADH-abhängige Reduktion von β-, γ- und δ-Ketoestern) zu erwarten.

### Beispiel 2 : Regulation der Ketoester-Reduktase

Die Regulation der Ketoester-Reduktase wurde untersucht. Dazu wurden Zellen auf unterschiedlichen C-Quellen (s. dazu Beispiel 1; 2) im 200 ml-Maßstab angezogen. Der Zeitpunkt, bei dem die Aktivität der Ketosäure-Reduktase maximal ist, wurde ermittelt und als Erntezeitpunkt festgelegt (s. Fig. 1).

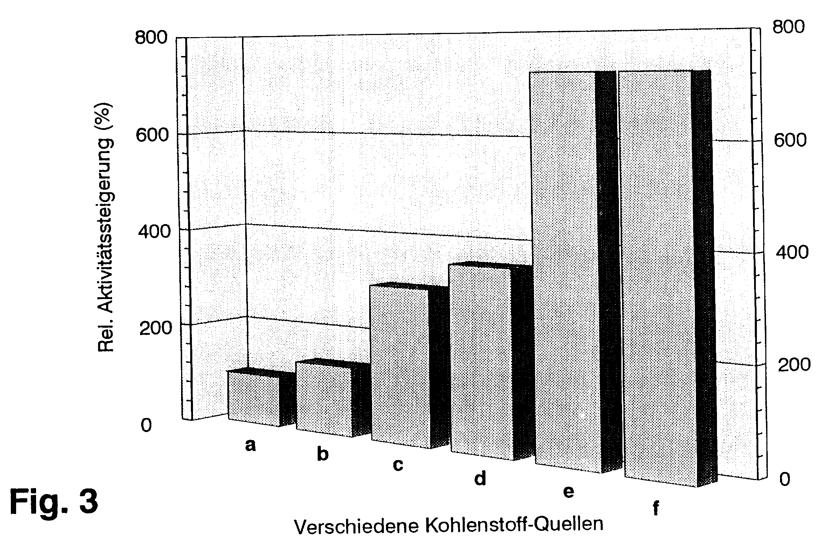

Die spezifische Aktivität der Ketoester-Reduktase für 5-Oxohexansäureethylester ist in Figur 3 in Abhängigkeit vom Wachstum auf unterschiedlichen C-Quellen dargestellt. Die Ketoester-Reduktase von *Candida parapsilosis* unterliegt der Katabolit-Repression. Unter dereprimierenden Bedingungen (0,8% Glucose im Medium) liegt die spezifische Aktivität des Enzyms etwa 36% höher, als unter reprimierenden Bedingungen (5,4% Glucose im Medium). Unter nicht-reprimierenden Bedingungen (2% Glycerin im Medium) ist die spezifische Aktivität etwa 300% höher, als unter reprimierenden Bedingungen. Bei Wachstum auf Dodecansäure wird die Aktivität der Ketoester-Reduktase von *Candida parapsilosis* auf über 700% gesteigert. Der Zusatz eines Ketoesters zum Medium (0,1%) bringt im Fall von Glycerin als C-Quelle eine Steigerung der spezifischen Aktivität auf 350%.

### Beispiel 3 : Charakterisierung der Ketoester-Reduktase

Für die folgenden Versuche wurde partiell gereinigtes Enzym (nach Q-Sepharose-Chromatographie, 67-fache Anreicherung) eingesetzt.

### A. pH-Abhängigkeit der Umsetzung

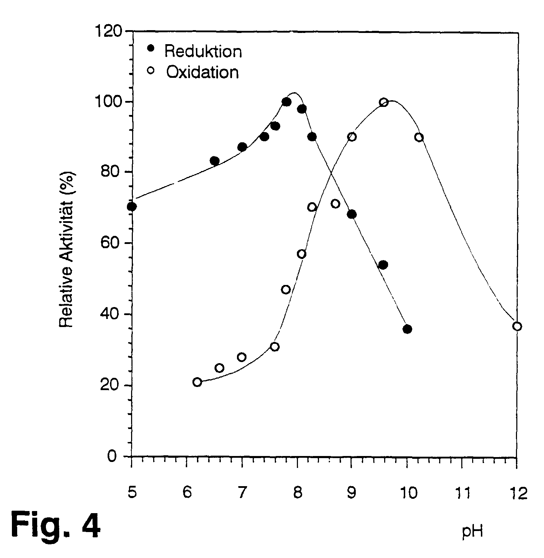

Die Abhängigkeit der Aktivität vom pH-Wert wurde bestimmt, indem 5-Oxohexansäureethylester (35 mM) mit NADH (0,2 mM) und 10 ml Enzymlösung bei unterschiedlichen pH-Werten (0,2 M Pufferlösung) gemischt wurden und die Aktivität bei 340 nm (30°C) photometrisch verfolgt wurde. Fig. 4 zeigt die erhaltenen Aktivitätswerte in Abhängigkeit vom pH-Wert, das Optimum für die 5-Oxohexansäureethylester-Reduktion liegt bei pH 7,9

In analoger Weise wurde das pH-Optimum für die Rückreaktion, die 5-Hydroxyhexansäureethylester-Oxidation, gemessen. (R/S)-5-Hydroxyhexansäureethylester (35 mM) wurde in 0,2 M Puffer mit pH-Werten zwischen 4 und 10 gelöst, 0,2 mM NADH und 10 µl Enzymlösung zugesetzt und die NADH-Bildungsrate photometrisch bei 340 nm verfolgt. Fig. 4 faßt die erhaltenen Werte zusammen, das pH-Optimum für die Oxidation liegt bei pH 9,5.

### B. Temperaturabhängigkeit der Reduktion

Zur Bestimmung des Temperaturoptimums der Reduktion wurde die Enzymaktivität zwischen 30 und 60°C gemessen. Der Testansatz enthielt :

| | |
|---|---|
| 5-Oxohexansäureethylester | 35 mM |
| NADH | 0,2 mM |
| Enzymlösung | 10 µl |

TEA-NaOH-Puffer, 0,1 M; pH 7,9

Die KERed aus *C. parapsilosis* hat ein Temperaturoptimum für die Reduktion zwischen 36 und 40°C.

### C. Temperaturoptimum der Oxidation

Zur Bestimmung des Temperaturoptimums der Oxidation wurde die Enzymaktivität zwischen 30 und 60°C gemessen. Der Testansatz enthielt :

| | |
|---|---|
| 5-Hydroxyhexansäureethylester | 35 mM |
| NAD | 0,5 mM |
| Enzymlösung | 10 µl |

TRIS-HCl-Puffer, 0.1 M, pH 9

Die KERed aus *C. parapsilosis* hat ein Temperaturoptimum für die Oxidation zwischen 50 und 56°C.

### D. Einfluß verschiedener Reagenzien auf die KERed

Der Einfluß der verschiedenen Metallkationen und Reagenzien auf die Enzymaktivität wurde untersucht, indem der unter Beispiel 3.B beschriebene Testansatz verwendet wurde.

Die Ergebnisse der Inhibitionsstudie sind in Tabelle 3 zusammengefaßt. Auffallend ist, daß das Enzym durch den Chelator o-Phenanthrolin relativ stark, durch EDTA hingegen praktisch nicht gehemmt wird. Offenbar besitzt die Ketoester-Reduktase ein essentielles Metallion, das durch o-Phenanthrolin entfernt wird, was wiederum zum Aktivitätsverlust führt. Die zunehmend starke Hemmung der Ketoester-Reduktase durch Thiol-Reagenzien wie N-Ethylmaleimid, Jodacetamid, 2,2'-Dithio-Bis(2-nitrobenzoat) und p-Hydroxymercuribenzoat weisen darauf hin, daß das Enzym mindestens eine essentielle SH-Gruppe enthält.

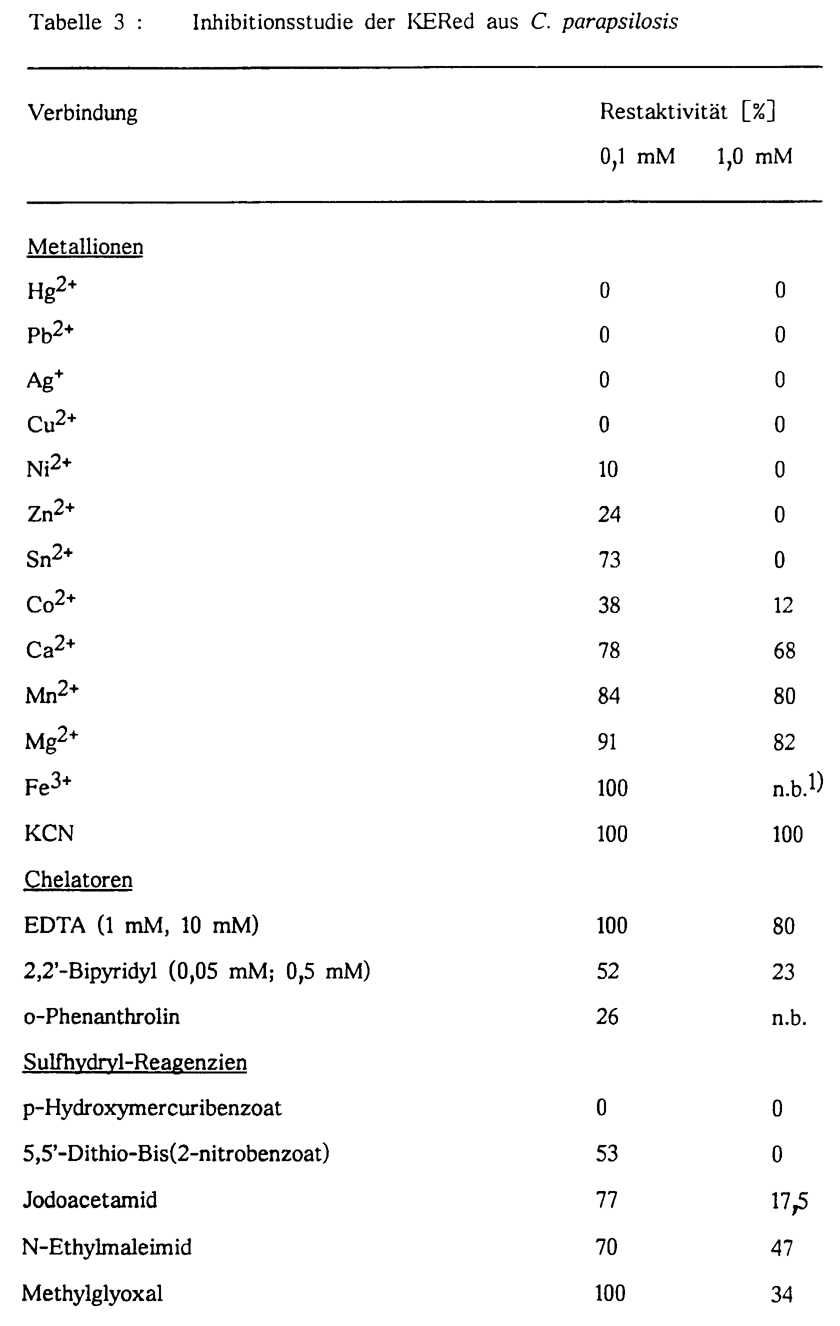

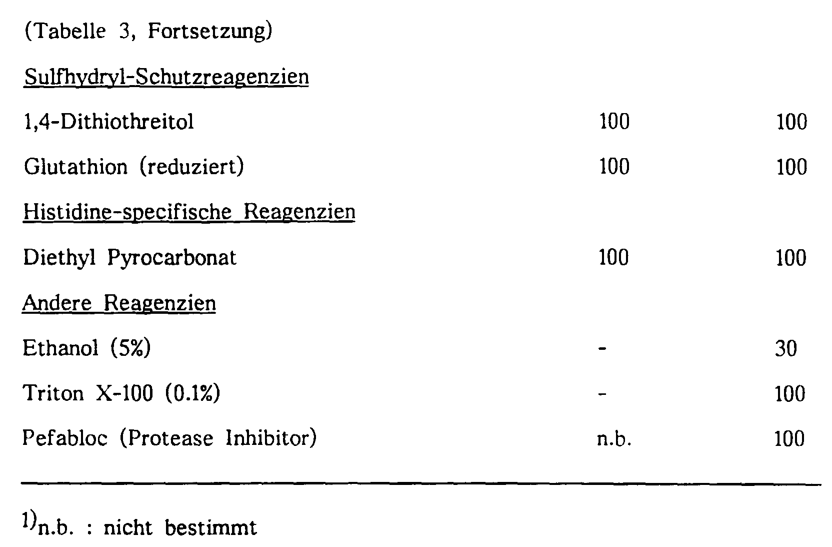

### E. Einfluß der Pufferkonzentration auf die Aktivität der KERed

Der Einfluß der Pufferkonzentration auf die Aktivität der Ketoester-Reduktase wurde getestet. Dazu wurde der in Beispiel 3.B angegebene Testansatz unter Variation der Pufferkonzentration verwendet.

Die Enzymaktivität ist maximal bei einer Molarität des Puffers von 0,1 M.

### F. Substratspektrum der KERed

Entsprechend Beispiel 3.B wurde an Stelle von 5-Oxohexansäureethylester eine Reihe aromatischer und aliphatischer Ketoester, Ketosäuren, Ketone, Diketone und Aldehyde daraufhin untersucht, ob sie enzymkatalysiert reduziert werden können. Dabei wurden die Verbindungen stets in 8 mM Endkonzentration zugesetzt. Die Ergebnisse sind in Tab. 4 zusammengestellt. Es zeigt sich, daß das Enzym eine Vielzahl aromatischer und aliphatischer Oxoverbindungen als Substrat akzeptiert.

Die Aktivität mit 5-Oxohexansäureethylester wurde willkürlich auf 100% gesetzt.

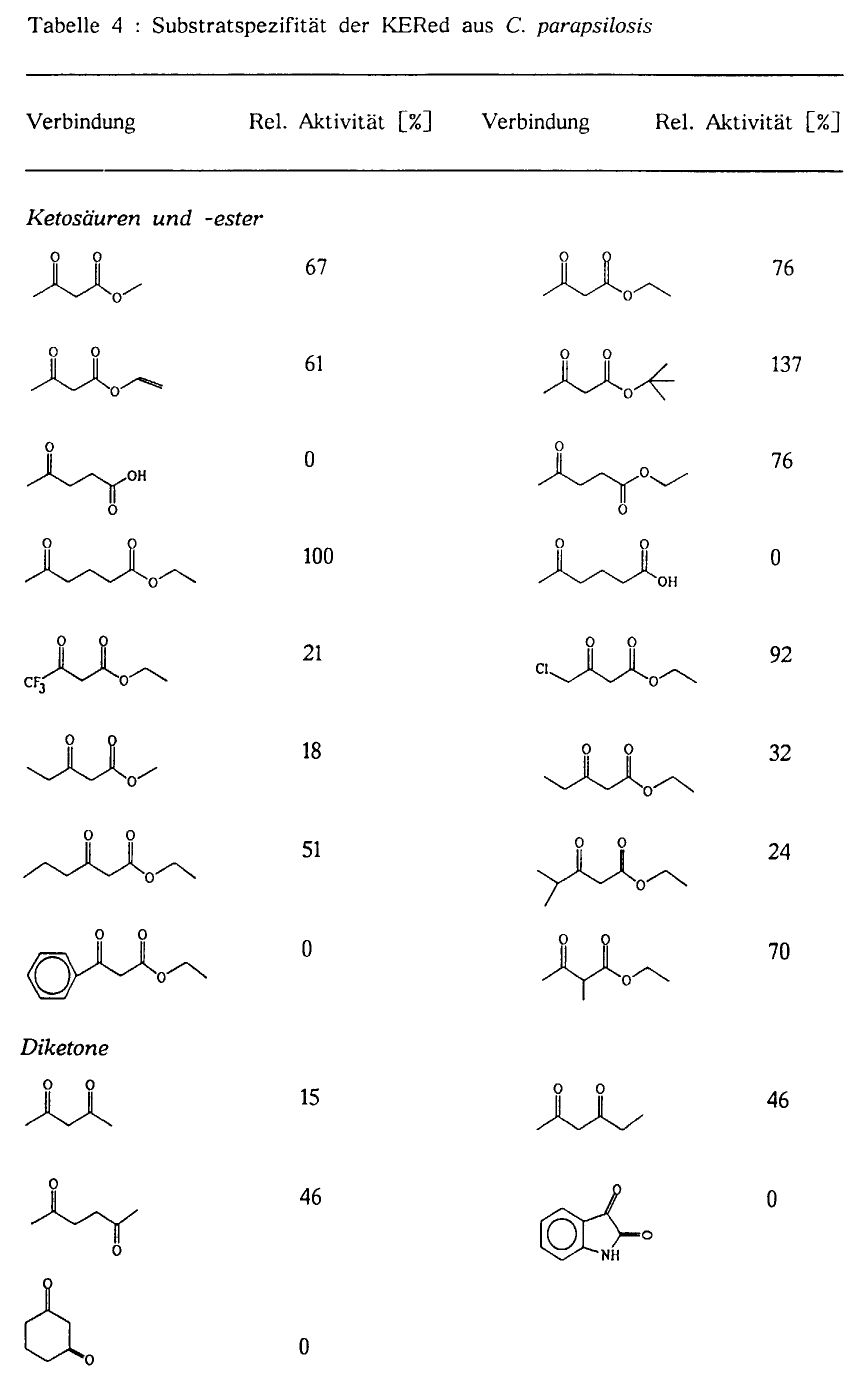

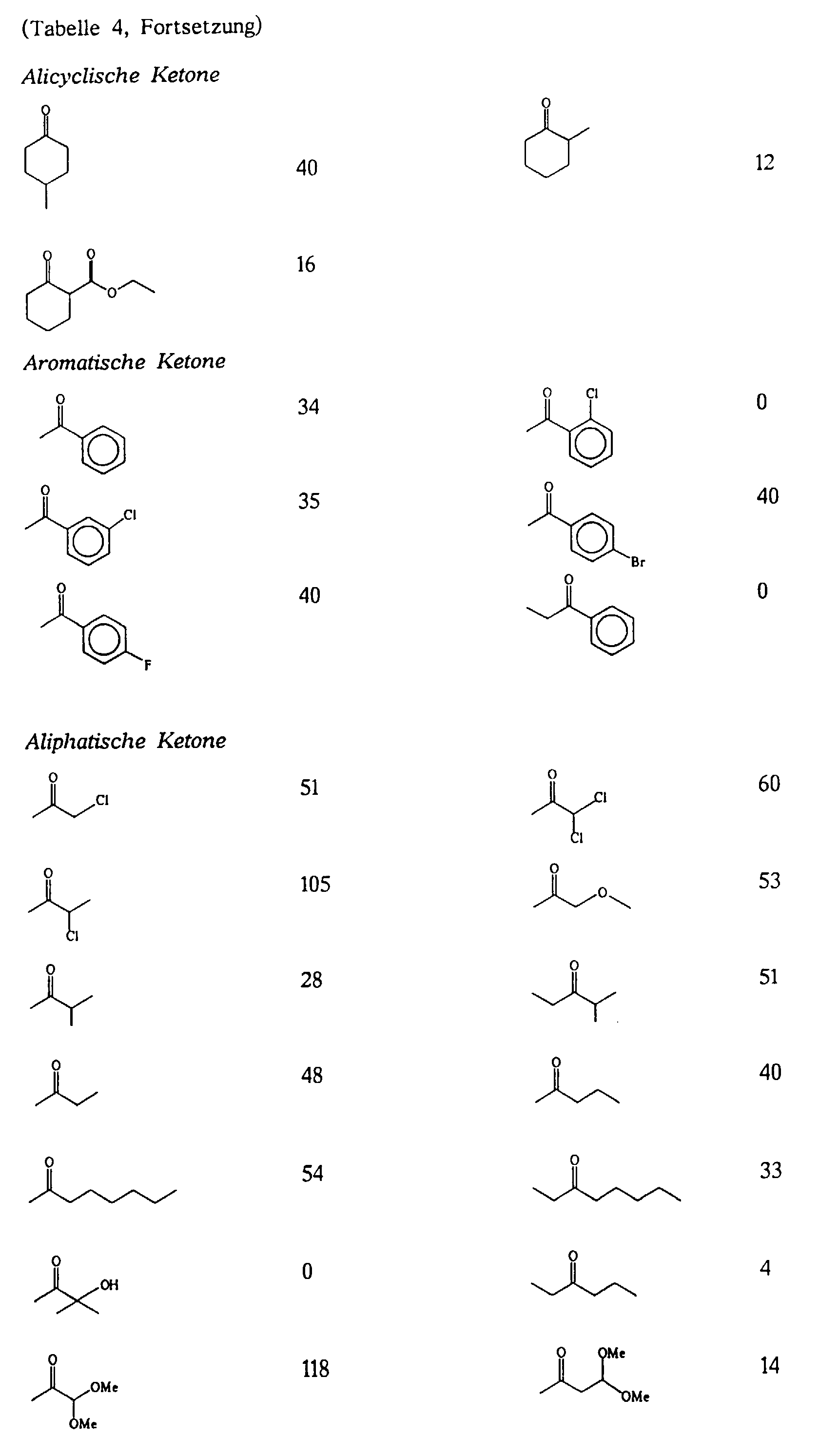

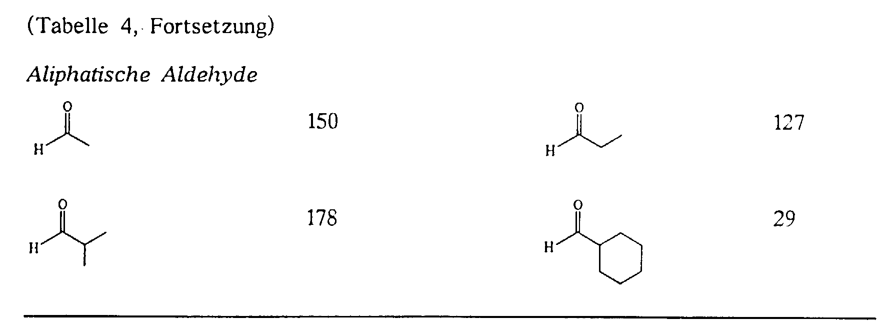

α-Ketocarbonsäuren und -ester und Allyl-Ketone werden nicht umgesetzt.

Wie man sieht, ist das Substratspektrum der Ketoester-Reduktase von erheblicher Breite, wobei außer β-, γ- und δ-Ketoestern 1,3- und 1,4-Diketone, ggf. substituierte insbes. halogenierte aliphatische, alicyclische und aromatische Ketone, Ketoacetale und Aldehyde akzeptiert werden.

### G. Kinetische Charakterisierung der KERed

Für ausgewählte Substrate wurden mit Hilfe des oben beschriebenen photometrischen Tests ausgewählte Substrate der KERed aus *C. parapsilosis* kinetisch vermessen. Die Ergebnisse der kinetischen Charakterisierung sind in Tabelle 5 zusammengefaßt.

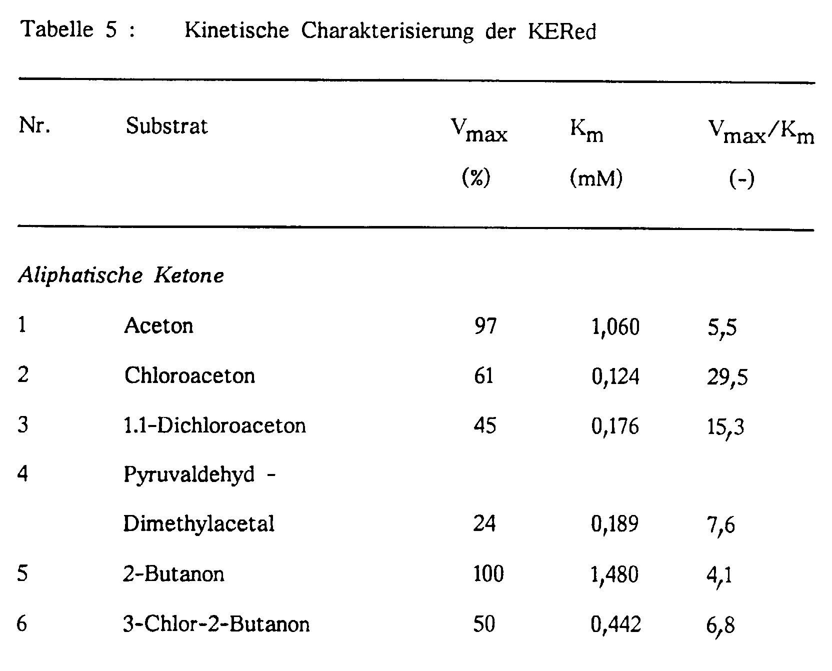

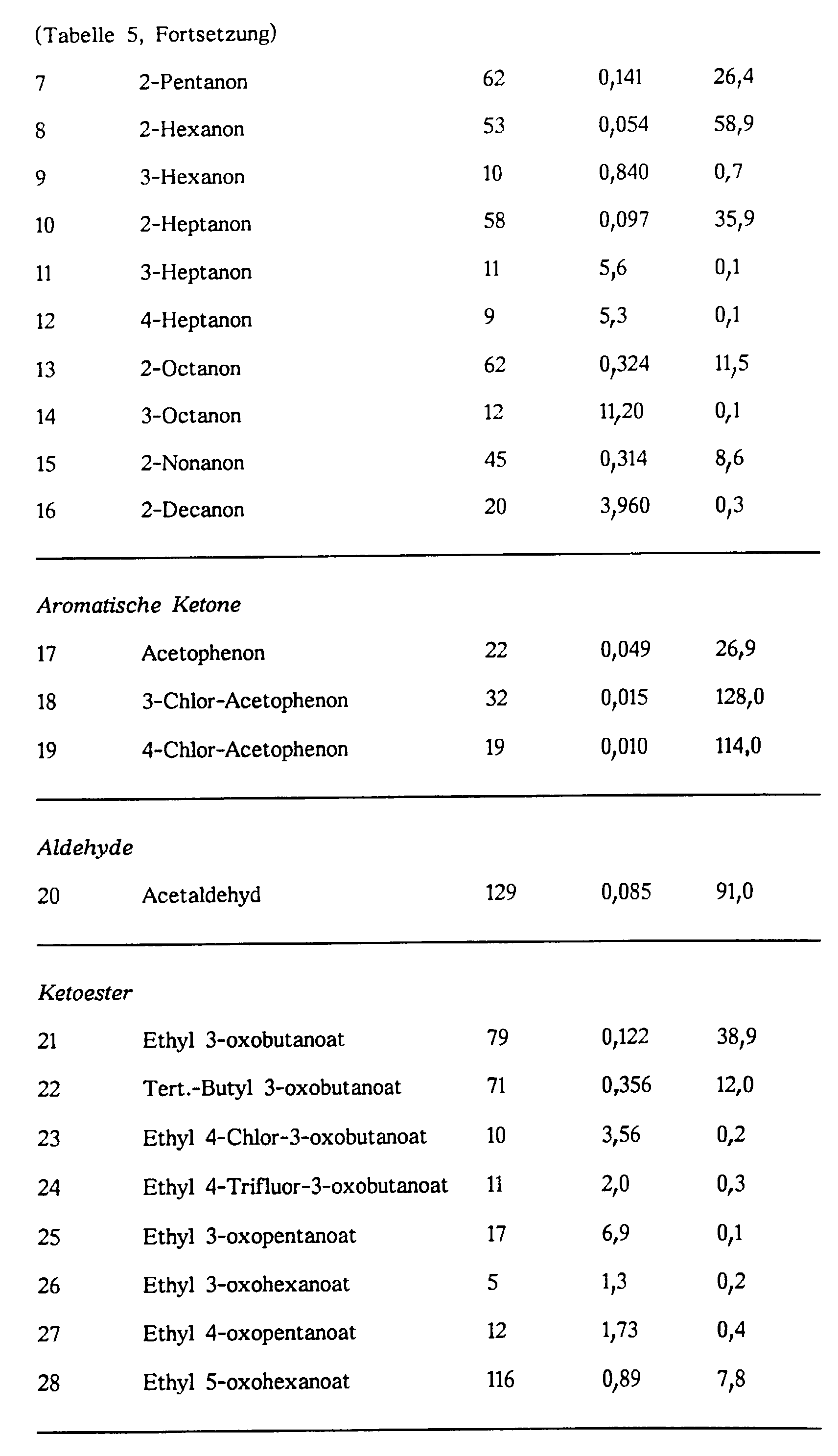

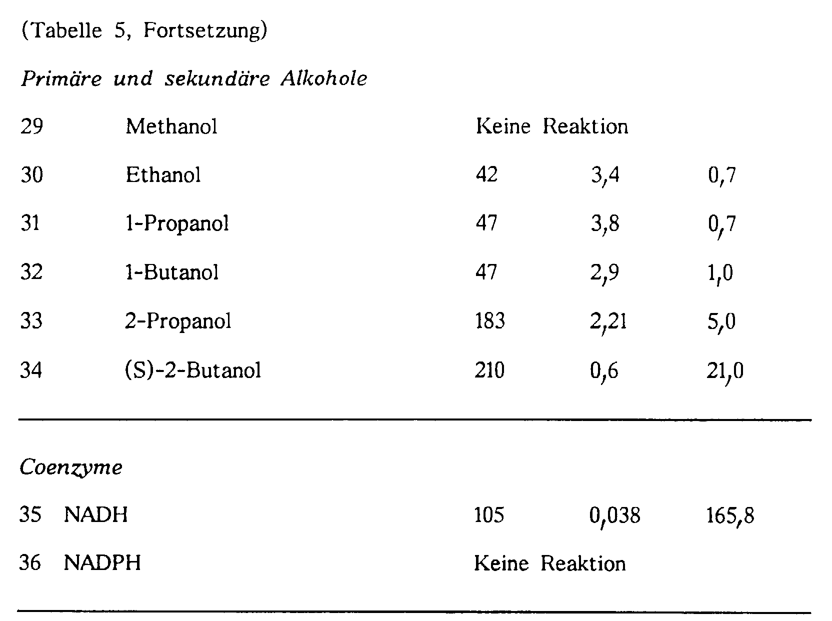

Alle Vₘₐₓ Werte sind bezogen auf 2-Butanon (6 U/mg, partiell gereinigtes Enzym). Substratinhibition wurde mit Chloroaceton (Kᵢₛ: 121.3 mM), 3-Hexanon (Kᵢₛ: 612 mM) und 2-Nonanon (Kᵢₛ: 24.9 mM) beobachtet. Generell lag der Standardfehler der maximalen Reaktionsgeschwindigkeiten und der Michaelis-Menten-Konstanten zwischen 5-10 Prozent der Werte.

Aus diesen Ergebnissen kann ein Modell für die Substratbindungsstelle der KERed abgeleitet werden. Durch dieses Modell ist eine Vorhersage möglich, ob ein bestimmtes potentielles Substrat von der KERed akzeptiert wird, oder nicht. In Fig. 5 ist das Modell der Substratbindungsstelle dargestellt.

Danach besteht die Substratbindungsstelle aus zwei Bereichen, die sich hinsichtlich Volumen und Hydrophobizität unterscheiden. Die kleine Tasche kann Methyl-, Ethyl- und Propylgruppen aufnehmen. Daneben werden auch substituierte Methylgruppen wie z.B. die Chlormethylgruppe akzeptiert. Die große Tasche kann lange und hydrophobe Reste binden. Wie aus Tabelle 5 hervorgeht, können dies aliphatische Reste bis C₈ sein (s. 2-Decanon). Die große Tasche kann eine Vielzahl auch sterisch anspruchsvoller Reste aufnehmen und bestimmt maßgeblich das außerordentlich breite Substratspektrum des Enzyms.

### H. Enzylnkatalysierte Herstellung von 3-Hydroxybuttecrsäuremethylester im Batch-Ansatz

Eine Lösung von 3-Oxobuttersäuremethylester (100 mM) wurde mit Ketoester-Reduktase (10 U) und Coenzym (0,1 mM) umgesetzt (100 ml Gesamtvolumen), wobei das Coenzym NADH durch Kopplung mit Natriumformiat (1 M) und Formiat-Dehydrogenase (40 U) regeneriert wurde. In regelmäßigen Abständen wurden Proben entnommen und mit Hilfe der Dünnschicht-Chromatographie (DC) auf gebildeten 3-Hydroxybuttersäuremethylester analysiert. Die Enantiomerenreinheit wurde über GC-Analyse bestimmt.

### Trennbedingungen der DC :

- Stationäre Phase :: Kieselgel 60 F254
- Mobile Phase :: Diethylether : Petrolether₆₀₋₈₀ = 2 : 1
- Laufstrecke :: 5 cm
- Tauchlösung :: 10%ige Molybdatophosphorsäure-Hydrat-Lösung in Ethanol (abs) mit 4 % HCl
- Verfahren :: Aufsteigende Chromatographie bei Kammersättigung
- Probevolumen :: 2-10 µl
- Laufverhalten :: Hydroxysäuren/-ester laufen weniger weit als Ketosäuren/-ester

Nachdem die Reaktionszeit von 36 Stunden abgelaufen war, wurde zur Bestimmung der Enantiomerenreinheit der Reaktionsansatz mit Chloroform extrahiert, daß ausgefallene Protein abgetrennt und die organische Phase mit Trifluoressigsäureanhydrid (TFAA) derivatisiert. Eine Probe wurde mittels chiraler Gaschromatographie analysiert.

### Trennbedingungen der DC :

- Säule :: Lipodes E (25m x 0.25 mm ID)
- Helium :: 0,8 bar
- Wasserstoff :: 0,5 bar
- Probenvolumen :: 1 µl
- T_{Säule} :: 80°C bzw. 90°C
- T_{Injektor} :: 206°C

### Retentionszeiten :

3-(R)-Hydroxybuttersäuremethylester 10,5 min
3-(S)-Hydroxybuttersäuremethylester 14,9 min

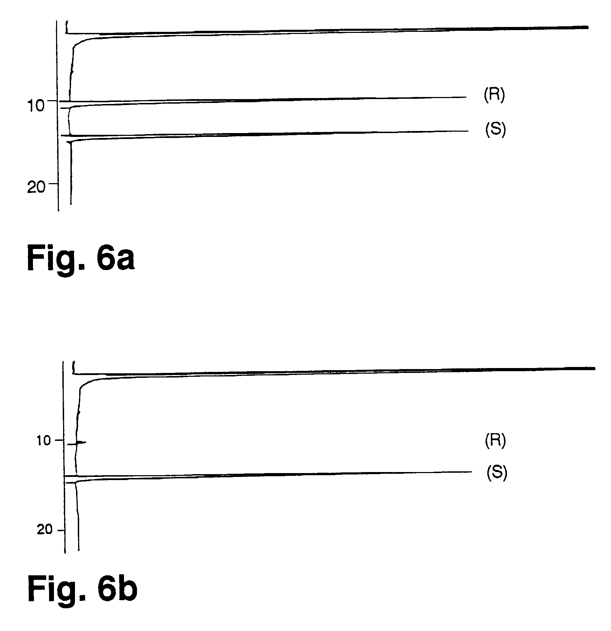

Die Elutionsreihenfolge der Enantiomeren auf der chiralen GC-Säule wurde mit reinen Enantiomeren von 3-Hydroxybuttersäuremethylester zu (*R*) vor (*S*) bestimmt. Die Ergebnisse der GC-Analyse sind in Fig. 6 dargestellt. Die Ketoester-Reduktase aus *Candida parapsilosis* setzt 3-Oxobuttersäuremethylester mit einem Enantiomerenüberschuß von 98% und einer Ausbeute von 95% zu (S)-3-Hydroxybuttersäuremethylester um.

### I. Enzymkatalysierte Herstellung von 5-Hydroxyhexansäureethylester im Batch-Ansatz

Analog zu Beispiel 3.H wurde 5-Oxohexansäureethylester enzymatisch im präparativen Maßstab reduziert. Proben wurde mittels chiraler Gaschromatographie analysiert (T_{S}: 90°C)

### Retentionszeiten :

5-(*R*)-Hydroxyhexansäureethylester 25,0 min

5-(*S*)-Hydroxyhexansäureethylester 26,9 min

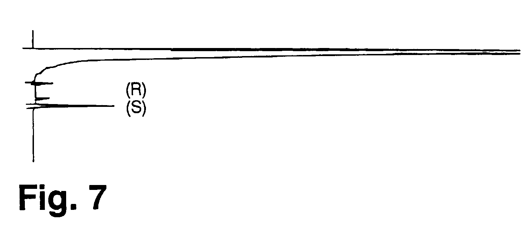

Die Ergebnisse der GC-Analyse sind in Fig. 7 dargestellt. Die Ketoester-Reduktase aus *Candida parapsilosis* setzt 5-Oxohexansäureethylester mit einem Enantiomerenüberschuß von 95% und einer Ausbeute von 96% zu (S)-5-Hydroxyhexansäureethylester um.

### J. Nachweis der Stereospezifität des Enzyms

Zum Nachweis der Stereospezifität des Enzyms und der Enantiomerenreinheit des Produkts wurden zwei Methoden verwendet, einerseits wurde enzymatisch hergestelltes Produkt mit einer chiralen Gaschromatographie, die (*R*)- und (*S*)-Hydroxyester voneinander trennen kann, untersucht, andererseits wurde mit käuflichen, reinen Isomeren von *(R)-* und (*S*)-Hydroxysäureestern sowie *(R)-* und (*S*)-Phenylethanol photometrisch die Oxidationsreaktion gemessen.

### 1. Chirale GC

Die Produkte der enzymatischen Umsetzungen sowie die käuflichen reinen Enantiomeren wurden mit Hilfe der Gaschromatographie an einer chiralen Phase analog zu Beispiel 3.H getrennt. Die Zuordnung der Peaks erfolgte durch Vergleich mit den käuflichen reinen Enantiomeren von 3-Hydroxybuttersäuremethylester. Die Elutionsreihenfolge (*R*) vor (*S*) gilt für die homologe Reihe der Hydroxyester. Fig. 6 zeigt, daß man durch enzymatische Umsetzung zu reinem (*S*)-Hydroxybuttersäuremethylester gelangt.

### 2. Rückreaktion mit (R)- bzw. (S)-Hydroxyverbindungen

Zum photometrischen Nachweis der Stereospezifität wurden folgende Testansätze durchgeführt :

| | | |
|---|---|---|
| I) | 35 mM | (*R*)-Hydroxybuttersäuremethylester |
| | 0.5 mM | NAD |
| | 10 µl | Enzymlösung |
| | TRIS-HCl-Puffer, pH 9, 0.1 M | |

| | | |
|---|---|---|
| II) | 35 mM | (S)-Hydroxybuttersäuremethylester |
| | 0.5 mM | NAD |
| | 10 µl | Enzymlösung |
| | TRIS-HCl-Puffer, pH 9, 0.1 M | |
| | Temperatur : 56°C | |

| | | |
|---|---|---|
| III) | 8 mM | (R)-Phenylethanol |
| | 0.5 mM | NAD |
| | 10 µl | Enzymlösung |
| | TRIS-HCl-Puffer, pH 9, 0.1 M | |

| | | |
|---|---|---|
| IV) | 8 mM | (S)-Phenylethanol |
| | 0.5 mM | NAD |
| | 10 µl | Enzymlösung |
| | TRIS-HCl-Puffer, pH 9, 0.1 M | |

Die Aktivitätsmessung erfolgte photometrisch bei 340 nm.

### Ergebnisse :

| | | | |
|---|---|---|---|
| Ansatz I : | 7 % | Ansatz II : | 100 % |
| Ansatz III : | 1,1 % | Ansatz IV: | 100 % |

Das Ergebnis zeigt deutlich, daß das Enzym hochspezifisch für (*S*)-3-Hydroxybuttersäuremethylester und (*S*)-Phenylethanol ist.

## Patentansprüche

1. Verfahren zur Gewinnung von Ketoester-Reduktase mit Befähigung zur NADH-abhängigen enzymatischen Umsetzung von β-, γ- und δ-Ketoestern zu den entsprechenden optisch aktiven β-, γ- und δ-Hydroxysäureestern,
**gekennzeichnet durch,**
Kultivierung von Stämmen von *Candida parapsilosis, Yarr*owinia *cellobiosa, Rhodococcus erythropolis* oder *Pseudomonas acidovorans* und Isolierung des von den Mikroorganismen gebildeten Enzyms aus dem Zell-Rohextrakt durch fraktionierte PEG-Fällung.

2. Verfahren nach Anspruch 1,
**gekennzeichnet durch**
nachfolgende chromatographische Auftrennung zur weiteren Aufreinigung des Enzyms.

3. Verfahren nach Anspruch 1 oder 2,
**dadurch gekennzeichnet,**
daß die Kultivierung der Stämme auf einem längerkettige(s) Alkansäure(n) bzw. Alkan(e) als C-Quelle enthaltenden Medium erfolgt.

4. Verfahren nach einem der vorangehenden Ansprüche,
**dadurch gekennzeichnet,**
daß der Stamm DSM 70 125 von Candida parapsilosis kultiviert wird.

5. Zur NADH-abhängigen enzymatischen Umsetzung von β-, γ- und δ-Ketoestern zu den entsprechenden optisch aktiven β-, γ- und δ-Hydroxysäureestern befähigte Ketoester-Reduktase, erhältlich nach einem der Ansprüche 1 bis 4.

6. Ketoester-Reduktase nach Anspruch 5,
**gekennzeichnet durch**
folgende Parameter in aufgereinigter Form:
- ein pH-Optimum für die Ketoester-Reduktion zwischen pH 7,8 und 8,0 und für die Rückreaktion um pH 9,5;
- ein Temperatur-Optimum zwischen 36 und 40°C für die Reduktion von Ketoestern, und von 50 bis 56°C für die Rückreaktion;
- rasche Desaktivierung durch Hg²⁺-, Pb²⁺-, Ag⁺-, Cu²⁺-, Ni²⁺-, Sn²⁺- und Co²⁺-lonen, starke Inhibierung durch p-Hydroxymercuribenzoat, 5,5'-Dithio-Bis(2-nitrobenzoat) und Jodacetamid sowie durch die Chelatoren 2,2'-Bipyridyl und o-Phenanthrolin und Stabilisierung durch SH-Schutzreagenzien wie Dithiothreitol;
- zuätzliche Befähigung zur Reduktion von aliphatischen, alicyclischen und aromatischen Ketonen, Diketonen, Ketalen und Aldehyden sowie zur Oxidation von primären und sekundären Alkoholen.

7. Verwendung der Ketoester-Reduktase nach Anspruch 5 oder 6, zur enzymatischen Reduktion von Oxo-Verbindungen unter Bildung optisch aktiver S-Hydroxy-Verbindungen in Gegenwart von NADH.

8. Verwendung von Ketoester-Reduktase nach Anspruch 5 oder 6, zur Herstellung optisch aktiver R-Hydroxy-Verbindungen, **dadurch gekennzeichnet,**
daß man von einem Racemat von R- und S-Hydroxy-Verbindungen ausgeht und das S-Enantiomere enzymatisch in Gegenwart von NAD in die Oxo-Verbindung überführt und abtrennt.

## Claims

1. A method of producing ketoester reductase with a capacity for the NADH-dependent enzymatic conversion of β-, γ- and δ-ketoesters into the corresponding optically active β-, γ-, and δ-hydroxyacid esters,
characterised by
the cultivation of strains of *Candida parapsilosis, Yarrowinia cellobiosa, Rhodococcus erythropolis* or *Pseudomonas acidovorans* and isolation of the enzyme formed by the microorganisms by fractional PEG precipitation from the crude cell extract.

2. A method according to claim 1,
characterised by
subsequent chromatographic separation for further purification of the enzyme.

3. A method according to claim I or 2,
characterised in that
cultivation of the strains is effected on a medium containing a longer chain alkanoic acid/acids or alkane/alkanes as the C source.

4. A method according to any one of the preceding claims,
characterised in that
strain DSM 70 125 of *Candida parapsilosis* is cultivated.

5. Ketoester reductase capable of the NADH-dependent enzymatic conversion of β-, γ-and δ-ketoesters into the corresponding optically active β-, γ- and δ-hydroxyacid esters, obtainable according to any one of claims I to 4.

6. Ketoester reductase according to claim 5,
characterised by
the following parameters in purified form:
- a pH optimum between pH 7.8 and 8.0 for ketoester reduction, and of around pH 9.5 for the back-reaction;
- a temperature optimum between 36 and 40°C for the reduction of ketoesters, and of 50 to 56°C for the back-reaction;
- rapid deactivation by Hg²⁺, Pb²⁺, Ag⁺, Cu²⁺, Ni²⁺, Sn²⁺ and Co²⁺ ions, pronounced inhibition by p-hydroxymercuribenzoate, 5,5'-dithio-bis(2-nitrobenzoate) and iodoacetamide and by the chelating agents 2,2'-bipyridyl and o-phenanthroline, and stabilisation by SH-protective reagents such as dithiothreitol;
- additional capacity for the reduction of aliphatic, alicyclic and aromatic ketones, diketones, ketals and aldehydes and for the oxidation of primary and secondary alcohols.

7. The use of the ketoester reductase according to claim 5 or 6 for the enzymatic reduction of oxo compounds with the formation of optically active S-hydroxy compounds in the presence of NADH.

8. The use of the ketoester reductase according to claim 5 or 6 for the production of optically active R-hydroxy compounds,
characterised in that
a racemate of R- and S-hydroxy compounds is used as the starting material, and the S-enantiomer is enzymatically converted into the oxo compound in the presence of NAD and is separated.

## Revendications

1. Procédé d'obtention d'une cétoester-réductase capable de transformer, par réaction enzymatique dépendante de NADH, des β-, γ- et δ-cétoesters en les esters β-, γ- et δ-hydroxyliques optiquement actifs correspondants, caractérisé en ce qu'on cultive des souches de *Candida parapsilosis,* de *Yarrowinia cellobiosa,* de *Rhodococcus erythropolis* ou de *Pseudomonas acidovorans* et on isole l'enzyme formée par les micro-organismes, de l'extrait cellulaire brut, par précipitation fractionnée avec un PEG.

2. Procédé selon la revendication 1, caractérisé en ce qu'on effectue une séparation chromatographique ultérieure pour purifier l'enzyme de façon supplémentaire.

3. Procédé selon la revendication 1 ou 2, caractérisé en ce qu'on effectue la culture les souches dans un milieu contenant un ou plusieurs acides alcanoïques ou alcanes à chaîne longue comme source de carbone.

4. Procédé selon l'une des revendications précédentes, caractérisé en ce qu'on cultive la souche DSM 70 125 de *Candida parapsilosis.*

5. Cétoester-réductase capable de transformer, par réaction enzymatique dépendante de NADH, des β-, γ- et δ-cétoesters en les esters β-, γ- et δ-hydroxyliques optiquement actifs correspondants, qu'on obtient selon l'une des revendications 1 à 4.

6. Cétoester-réductase selon la revendication 5, caractérisée par les paramètres suivants à l'état purifié :
- une valeur optimale de pH comprise entre pH 7,8 et 8,0 pour la réduction des cétoesters et d'environ pH 9,5 pour la réaction en retour ;
- une température optimale comprise entre 36 et 40°C pour la réduction des cétoesters, et de 50 à 56°C pour la réaction en retour ;
- une désactivation rapide par les ions Hg²⁺, Pb²⁺, Ag⁺, Cu²⁺, Ni²⁺, Sn²⁺ et Co²⁺, une forte inhibition par le p-hydroxymercuribenzoate, le 5,5'-dithio-bis(2-nitrobenzoate) et l'iodo-acétamide, ainsi que par les chélateurs 2,2'-bipyridyle et o-phénanthroline, et stabilisation par des réactifs protecteurs des groupements SH, comme le dithiothréitol ;
- une aptitude supplémentaire à réduire les cétones, dicétones, cétals et aldéhydes, aliphatiques, alicycliques et aromatiques, ainsi qu'à oxyder les alcools primaires et les alcools secondaires.

7. Utilisation de la cétoester-réductase selon la revendication 5 ou 6 pour la réduction enzymatique de composés oxo avec formation de composés S-hydroxy optiquement actifs, en présence de NADH.

8. Utilisation d'une cétoester-réductase selon la revendication 5 ou 6 pour la préparation de composés R-hydroxy optiquement actifs, caractérisée en ce qu'on part d'un racémate de composés R- et S-hydroxy, on transforme l'énantiomère S en composé oxo, par voie enzymatique, en présence de NAD, et on le sépare.
